# EUROPEAN PATENT APPLICATION

(11) **EP 1 731 615 A1**
(43) Date of publication of application: **13.12.2006**
(21) Application number: 05719362.5
(22) Date of filing: 21.02.2005
(51) Int. Cl.: C12N 15/87, A61K 31/7088, A61K 47/48, A61P 43/00, A61P 35/00, A61P 35/02

(54) **CYTOPLASMIC LOCALIZATION DNA AND RNA**

(30) Priority: 20.02.2004 JP 2004045488; 30.04.2004 JP 2004136228
(71) Applicant: National Institute of Advanced Industrial Science and Technology, Tokyo 100-8921 (JP); Kitakyushu Foundation for the Advancement of Industry, Science and Technology, Kitakyushu-shi, Fukuoka 804-0003 (JP)
(72) Inventor: OBA, Hideki, c/o Nat. Inst. Adv. Ind. Sci. & Tech., Tosu-shi, Saga 841-0052 (JP); FUJII, Masayuki, Sch.Humanity-Oriented Sci. & Eng., Iizuka-shi, Fukuoka 820-0011 (JP)
(74) Representative: Baldock, Sharon Claire
(86) International application number: PCT/JP2005/002743
(87) International publication number: WO 2005/080582

(57) **Abstract**

It is intended to provide a modified DNA or RNA, the cytoplasmic localization of which has been established, and an siRNA, which is localized in the cytoplasm, shows a high activity and, therefore, is appropriately usable as a genetic drug, by using a means generally applicable to DNAs of various types regardless of original tissues. A cytoplasmic localization DNA or RNA modified with a peptide can be constructed by modifying a DNA fragment with an active hydrogen-containing group on a solid support, fusing a peptide having an active hydrogen-containing group therewith and then removing from the solid support. On the other hand, a cytoplasmic localization siRNA can be obtained by introducing chemical modification group(s) into the 5'-terminus of at least one of the sense chain and the antisense chain constituting the double-strand, or a dangling end of the antisense chain, or both of them.

## Description

### Technical Field

The present invention relates to a novel DNA and RNA localized in the cytoplasm, which are improved in their cytoplasmic permeability and are capable of being selectively localized in the cytoplasm by introducing a chemical modification group such as nuclear export signal peptides or membrane fusion peptides derived from proteins of various types, or polyamines into a DNA or RNA via covalent bond.

### Background Art

Proteins that act in the nuclei of organisms contain a portion serving as a mark for transfer from the cytoplasm to the nucleus, that is, a nuclear localization signal peptide, and this nuclear localization signal peptide is specific to each of nucleoproteins of various types. There exist a large number of factors that recognize the signal and transport the nucleoproteins from the cytoplasm to the nucleus. This achieves protein supply for life support.

On the other hand, proteins having a unique amino acid sequence designated as a nuclear export signal (hereinafter, referred to as NES) bind to transport proteins and move from the nucleus to the cytoplasm. Alternatively, membrane fusion proteins are proteins derived from viruses of various types that work for transport across the cell membrane.

As described above, the cells of organisms synthesize nuclei and proteins performing the preservation of genes and the sterminusing of genetic information and constantly exchange information in the cytoplasm serving as a setting for receiving extracellular stimuli, thereby maintaining vital activities.

Recently, so-called genetic medicines, which directly act on genes (DNAs or RNAs) and inhibit causative gene expression of disease to treat the disease, have received attention in that they are able to directly act on DNAs or RNAs, the origins of genetic information, and radically treat disease. For example, a hepatocyte-specific genetic medicine consisting of a vector containing a sugar-modified peptide derivative capable of hepatocyte-specific gene transfer, and a DNA or antisense DNA has been proposed as such a genetic medicine (see Japanese Patent Laid-Open No. 11-290073).

This genetic medicine must act on only one target from among human genes as many as 22000 and therefore requires a molecule that selectively binds to only one site of the human genome sequence consisting of 3.07 billion base pairs. The application of a short-chain DNA or RNA having this function, that is, a so-called oligo DNA or RNA, to medicines has been studied.

As a result, an anti-gene method that targets DNAs, an antisense method that targets mRNAs, a DNA enzyme method that targets RNAs by using DNAs having the function of degrading the RNAs, and an siRNA method that targets RNAs by using RNA interference (RNAi) triggered by double-stranded RNAs have already been developed. An important challenge to all of these methods is to create oligo DNA or RNA molecules having properties as medicines.

On the other hand, the RNA interference exhibits much stronger ability to control gene expression than those exhibited by antisense RNAs or ribozymes and as such, is increasingly expected as a tool for genetic medicines or the elucidation of gene functions.

However, siRNAs are difficult to introduce into cells and exhibit low nuclease resistance in cells. Thus, they do not produce stable effects and cannot therefore be in actual use.

Most of these genetic medicines are known to exert higher efficacy in the cytoplasm than in the cell nucleus. Therefore, if the cytoplasmic localization of the genetic medicines could be achieved, excellent genetic medicines should be obtained. However, such genetic medicines have been unknown so far.

From these points of view, the present inventors developed a method for synthesizing a DNA or RNA conjugate capable of being localized in the cytoplasm and however, fell short of the demonstration of its intracellular localization (Japanese Patent Laid-Open No. 2004-275140, The Society of Polymer Science, Japan, Proceedings Vol. 51, No. 14, pp. 3658, 2002, and T. Kubo et al., Organic Lett., 5, 2623-2626, 2003).

### Disclosure of the Invention

An object of the present invention is to provide a modified DNA or RNA, the cytoplasmic localization of which has been established, and a novel siRNA, which is not degraded by enzymes in cells by virtue of its enhanced enzyme resistance, is localized in the cytoplasm, shows a high activity and, therefore, is appropriately usable as a genetic medicine, by using a means generally applicable to DNAs or RNAs of various types regardless of original tissues.

The present inventors have conducted studies in various ways for obtaining a DNA or RNA capable of being localized in the cytoplasm and have consequently found that a DNA or RNA can be localized in the cytoplasm by forming a complex between an intracellularly residing protein and RanGTP and modifying the DNA or RNA with this complex and a peptide for transfer from the nucleus to the cytoplasm or a chemical modification group. Moreover, the present inventors have conducted studies in various ways for achieving an siRNA capable of being introduced into cells and enhanced in its enzyme resistance in cells or capable of being localized in the cytoplasm and have consequently found that an siRNA can be introduced into cells and enhanced in its enzyme resistance in cells by introducing, into the siRNA, chemical modification groups capable of imparting cationicity or fat solubility thereto, and can be localized in the cytoplasm by using peptides bonded together as this chemical modification group. Based on these findings, the present invention has been completed.

Namely, the present invention provides: a DNA or RNA localized in the cytoplasm having the 3'-terminus or 5'-terminus chemically modified with a group represented by the formula
-PO(OH)-O-CH₂CH₂OCH₂CH₂NH-CO-peptide or
-O-CO-NH-CH₂CH₂NHCONH(CH₂)₆NH-CO-NH-peptide; a DNA or RNA localized in the cytoplasm characterized by being constructed by modifying a DNA or RNA fragment with an active hydrogen-containing group on a solid support, fusing an NES peptide or membrane fusion peptide having an active hydrogen-containing group therewith via a bifunctional linker and then removing from the solid support; a siRNA localized in the cytoplasm characterized in that a chemical modification group is introduced into the 5'-terminus of at least one of the sense strand and the antisense strand constituting the double-strand or a dangling end of the antisense strand, or both; and a siRNA localized in the cytoplasm characterized in that at least one of the sense strand and the antisense strand contains a chemical modification group at a non-terminal position.

In the present invention, a DNA or RNA having the 3'-terminus or 5'-terminus to be modified with a group represented by the formula
-PO(OH)-O-CH₂CH₂OCH₂CH₂NH-CO-peptide or
-O-CO-NH-CH₂CH₂NHCONH(CH₂)₆NH-CO-NH-peptide
may be derived from any origin and can arbitrarily be selected from among those collected from a variety of tissues in organisms according to the purpose of the usage. Preferably, the DNA or RNA is an oligo DNA or RNA of approximately 10 to 30 bases.

Examples of the peptide used include nuclear export signal peptides (hereinafter, abbreviated to NES peptides) such as HIV-1 Rev (SEQ ID NO: 1 in Sequence Listing), PKIα (SEQ ID NO: 2 in Sequence Listing), MAPKK (SEQ ID NO: 3 in Sequence Listing), and Dsk-1 (SEQ ID NO: 4 in Sequence Listing), and membrane fusion peptides such as an HIV-1 tat C-terminal membrane fusion peptide (SEQ ID NO: 5 in Sequence Listing), a gp-41 membrane fusion peptide (SEQ ID NO: 6 in Sequence Listing), an artificially designed amphipathic α-helical peptide (SEQ ID NO: 7 in Sequence Listing), and an artificially designed amphipathic β-sheet peptide (SEQ ID NO: 8 in Sequence Listing). Other peptides can also be used.

Examples of known methods for modifying the DNA or RNA with such a peptide or the like include a method by which the DNA or RNA is fused with the peptide in a liquid phase via γ-maleinimidobutyloxysuccinimide used as a linker, a method by which the DNA or RNA is fused with the peptide in a liquid phase via iodoacetoxysuccinimide used as a linker, a method by which they are conjugated in a liquid phase, and a solid-phase fragment condensation method. Any of these methods may be used. Particularly, the solid-phase fragment condensation method is preferable.

This method is a method by which a bifunctional linker is reacted with an active hydrogen-containing group, for example, an amino, carboxyl, thiol, or hydroxyl group, of a DNA fragment on a solid support such as porous glass, controlled pore glass (CPG), and polyethylene glycol/polystyrene, followed by the condensation of the peptide therewith.

The bifunctional linker used in this method is a compound having two functional groups capable of forming stable bond with the active hydrogen-containing group through their reaction.

Examples of such a compound include compounds represented by the following (1) to (8):
(1) S-(2-pyridyldithio)cysteamine 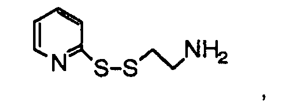
(2) N-succinimidyl 3-(2-pyridyldithio)propionate 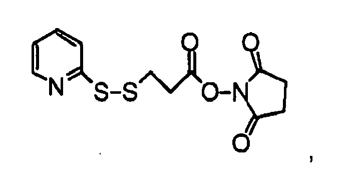
(3) iodoacetoxysuccinimide 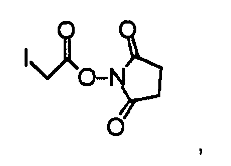
(4) dithioisocyanatoalkane

   SNC-(CH₂)ₙ-NCS,
(5) diisocyanatoalkane

   ONC-(CH₂)ₙ-NCO,

   wherein n represents an integer of 1 to 10,
(6) 2-(2-pyridyldithio)ethyl isocyanate 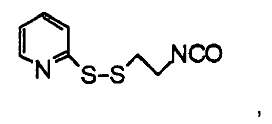
(7) N-(4-maleimidobutyryloxy)succinimide 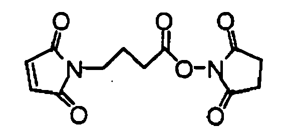 and
(8) N-4-maleimidobutyric acid

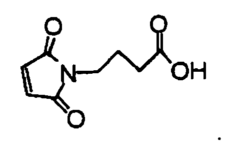

The reaction between this DNA fragment or the like and the bifunctional linker is performed by adding the bifunctional linker dissolved in, for example, an acetonitrile or dimethylformamide solution, to the DNA fragment or the like on the solid support, followed by stirring at 10 to 40 °C for 2 to 10 hours. A bifunctional linker concentration in this solution is preferably 0.1 to 1 mol/l. After the termination of the reaction, the solid support is well washed with the solvent to remove impurities.

Next, the reaction of the peptide with the condensation product thus obtained between the DNA fragment or the like and the bifunctional linker is performed by adding the peptide dissolved in an organic solvent, for example, acetonitrile or dimethylformamide, to the condensation product still held on the solid support, followed by stirring at 10 to 40 °C for 2 to 10 hours. After the termination of the reaction, the solid support is washed with the same solvent to remove impurities, thereby obtaining a DNA or RNA conjugate bound with the solid support.

Subsequently, the DNA or RNA conjugate is removed from the solid support by alkali treatment and purified by chromatography or the like to obtain the desired DNA or RNA localized in the cytoplasm at a yield of 2 to 50%.

The alkali used in this procedure is preferably concentrated ammonia water or an aqueous solution of 0.5 M sodium carbonate.

Next, a reaction formula is shown as one example of a method for modifying the 5'-position of the DNA with the NES peptide. 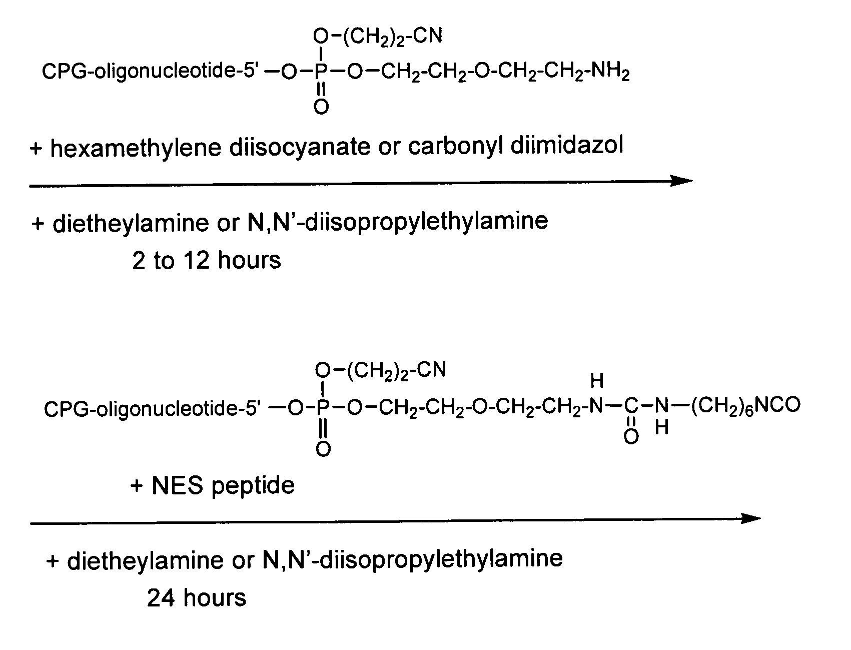 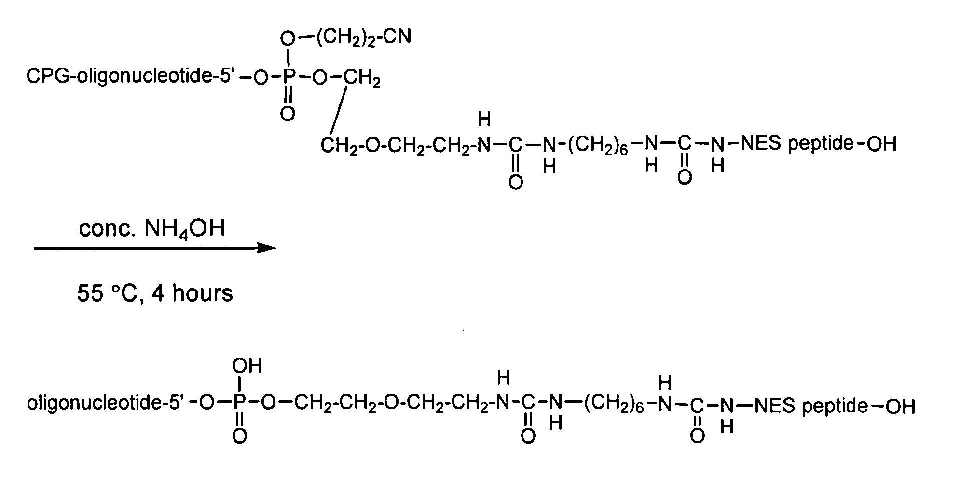 (CPG in the formula represents controlled pore glass.)

In this example, the 5'-position of the oligopeptide is modified. The 3'-position thereof can also be modified in the same way by binding the 5'-position of the DNA fragment to the solid support.

A preferable unit for linking the oligopeptide and the DNA fragment other than the unit formed by the bifunctional linker is (9) a group represented by the formula

-PO(OH)-O-CH₂CH₂OCH₂CH₂NH-CO-

or (10) a group represented by the formula

-O-CO-NH-CH₂CH₂NHCONH(CH₂)₆NH-CO-NH-.

The introducibility into cells of the DNA localized in the cytoplasm thus obtained can be confirmed by fluorescently labeling the N-terminus of the oligopeptide with fluorescein isothiocyanate or the like and then introducing it into a given cell, which is in turn cultured and examined by use of flow cytometry and a fluorescence and laser scanning confocal microscope.

Of the RNAs of the present invention, the siRNAs are a siRNA localized in the cytoplasm characterized in that a chemical modification group is introduced into the 5'-terminus of at least one of the sense strand and the antisense strand constituting the double-strand or a dangling end of the antisense strand, or both, and a siRNA localized in the cytoplasm characterized in that at least one of the sense strand and the antisense strand contains a chemical modification group at a non-terminal position.

In this context, the dangling end means a portion non-complementary with the anti strand of the antisense strand constituting the double-strand, i.e. a single-stranded portion.

In the siRNA localized in the cytoplasm of the present invention, a chemical modification group is introduced into the 5'-terminus of at least one of the sense strand and the antisense strand constituting it or a dangling end of the antisense strand, or both.

Examples of such a siRNA localized in the cytoplasm can include a siRNA localized in the cytoplasm composed of the sense strand represented by the formula
5'-SEQ ID NO: 9 in Sequence Listing-3'
and the antisense strand represented by the formula
5'-SEQ ID NO: 10 in Sequence Listing-3',
wherein a chemical modification group is introduced into only the 5'-terminus of the sense strand, wherein a chemical modification group is introduced into only the 5'-terminus of the antisense strand, or wherein chemical modification groups are introduced into the 5'-termini of both the sense strand and the antisense strand, and
a siRNA localized in the cytoplasm composed of the sense strand represented by the formula
5'-SEQ ID NO: 9 in Sequence Listing-3'
and the antisense strand represented by the formula
5'-SEQ ID NO: 10 in Sequence Listing-3',
wherein chemical modification groups are introduced into both of the 5'-terminus of the sense strand and a dangling end of the antisense strand, or wherein a chemical modification group is introduced into only a dangling end of the antisense strand.

Examples of the chemical modification group introduced in this procedure can include a group capable of imparting water solubility such as an ether residue of alkylene glycol or polyalkylene glycol and hydroxyalkyl amine represented by the general formula

-(O-A)ₙ-NH₂ (I)

wherein A represents an alkylene group, and n represents an integer of 1 or higher, and
a residue of an alkylene diamine adduct of carboxylic acid amide with a cyclic organic base represented by the general formula 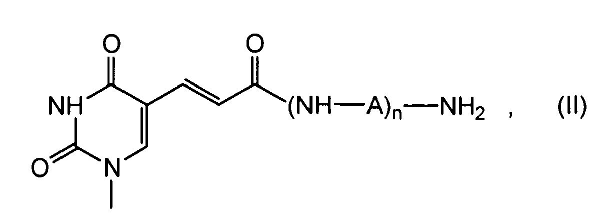 wherein A and n represent the same as above.

Among them, a compound wherein A represents an ethylene group, and n represents an integer of 1 or 2 is particularly preferable.

Next, examples of the siRNA localized in the cytoplasm wherein at least one of the sense strand and the antisense strand contains a chemical modification group at a non-terminal position include a siRNA localized in the cytoplasm composed of the sense strand represented by the formula
5'-SEQ ID NO: 9 in Sequence Listing-3'
and the antisense strand represented by the formula
5'-SEQ ID NO: 10 in Sequence Listing-3',
wherein a chemical modification group is substituted for only t at the 2nd position from the 3'-terminus of the sense strand, wherein a chemical modification group is substituted for only a dangling end of the antisense strand, that is, t at the 2nd position from the 3'-terminus of the antisense strand, wherein chemical modification groups are respectively substituted for t at the 2nd position from the 3'-terminus of the sense strand and a dangling end of the antisense strand, that is, t at the 2nd position from the 3'-terminus of the antisense strand, wherein chemical modification groups are substituted for u at the 6th and 15th positions from the 5'-terminus of the sense strand, wherein chemical modification groups are substituted for u at the 5th, 7th, 10th, and 15th positions from the 3'-terminus of the antisense strand, or wherein chemical modification groups are substituted for u at the 6th and 15th positions from the 5'-terminus of the sense strand and u at the 5th, 7th, 10th, and 15th positions from the 3'-terminus of the antisense strand.

The number and binding order of bases in these sense and antisense strands are not particularly limited and can arbitrarily be selected from among heretofore known siRNA structures. Moreover, the structure of the chemical modification group is not particularly limited, and its size, functional group types, and so on can arbitrarily be selected.

The siRNA having the chemical modification group thus introduced is enhanced in its enzyme resistance in cells, and no cytotoxicity is observed therein.

When an NES peptide bonded via the bifunctional linker is used as a chemical modification group, examples of this NES peptide include HIV-1 Rev (SEQ ID NO: 1 in Sequence Listing), PKIα (SEQ ID NO: 2 in Sequence Listing), MAPKK (SEQ ID NO: 3 in Sequence Listing), Dsk-1 (SEQ ID NO: 4 in Sequence Listing), and TFIIIA (SEQ ID NO: 11 in Sequence Listing). Other NES peptides can also be used.

Examples of peptides other than the NES peptide include an HIV-1 tat C-terminal membrane fusion peptide (SEQ ID NO: 5 in Sequence Listing), a gp-41 membrane fusion peptide (SEQ ID NO: 6 in Sequence Listing), an SV40 T antigen nuclear localization signal peptide (SEQ ID NO: 12 in Sequence Listing), an HIV-1 tat nuclear localization signal peptide (SEQ ID NO: 13 in Sequence Listing), an artificially designed amphipathic α-helical peptide (SEQ ID NO: 7 in Sequence Listing), an artificially designed amphipathic β-sheet peptide (SEQ ID NO: 8 in Sequence Listing), an artificially designed amphipathic β-sheet peptide (SEQ ID NO: 14 in Sequence Listing), and an artificially designed amphipathic β-sheet peptide (SEQ ID NO: 15 in Sequence Listing).

Furthermore, polyamine such as spermine, spermidine, glucosamine, and galactosamine can also be introduced. The introduction of such polyamine improves enzyme resistance in cells.

To introduce the peptide or the polyamine into an siRNA via the bifunctional linker, the sense strand or antisense strand of the siRNA is first reacted with the bifunctional linker. This reaction is performed according to the solid-phase fragment condensation method by initially fusing the sense strand or antisense strand onto a solid support such as porous glass, controlled pore glass (CPG), and polyethylene glycol/polystyrene and causing a reaction of its active hydrogen-containing group, for example, an amino, carboxyl, thiol, or hydroxyl group, with the bifunctional linker.

A solvent used in this procedure is preferably a polar solvent such as acetonitrile, dimethylformamide, dimethylacetamide, and dimethylsulfoxide. A reaction temperature in this procedure is 10 to 40 °C. A reaction time differs depending on the type of the bifunctional linker reacted, the type of the peptide or the polyamine reacted, a reaction temperature, and so on and is approximately 2 to 10 hours. An appropriate bifunctional linker concentration in this solvent is 0.1 to 1 mol/l. After the termination of the reaction, the solid support is well washed with the same solvent to remove impurities.

Next, the reaction of the peptide or the polyamine with the condensation product thus obtained between the sense strand or antisense strand of the siRNA and the bifunctional linker is performed by adding the peptide or the polyamine dissolved in an organic solvent, for example, acetonitrile or dimethylformamide, to the condensation product still held on the solid support, followed by stirring at 10 to 40 °C for 2 to 10 hours. After the termination of the reaction, the solid support is washed with the same solvent to remove impurities, thereby obtaining the siRNA bound with the solid support.

Subsequently, the siRNA is removed from the solid support by alkali treatment and purified by chromatography or the like to obtain the desired siRNA localized in the cytoplasm at a yield of 2 to 50%.

The alkali used in this procedure is preferably concentrated ammonia water or an aqueous solution of 0.5 M sodium carbonate.

Examples of the bifunctional linker include compounds (1) to (8) shown above. It is particularly preferable to use a compound capable of introducing the chemical modification group represented by the general formula (I) or (II). Examples of such a compound include amine represented by the formula

HO-CH₂-CH₂-O-CH₂-CH₂-NH₂,

and amine represented by the formula

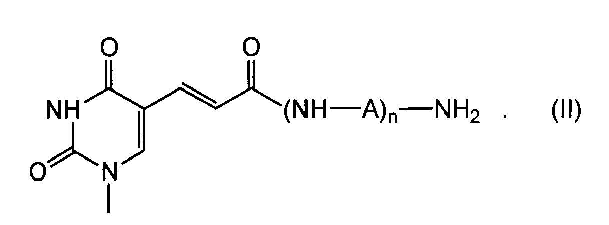

When the sense strand or antisense strand is reacted with such a bifunctional linker and then with peptides or polyamines of various types, a chemical modification group represented by the general formula 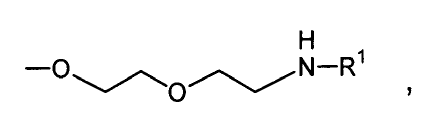 wherein R¹ represents a peptide such as an NES peptide, membrane fusion peptide, and nuclear localization signal peptide or polyamine such as spermine, spermidine, glucosamine, galactosamine, tris(2-aminoethyl)amine, and triethylenetetramine,
or the general formula 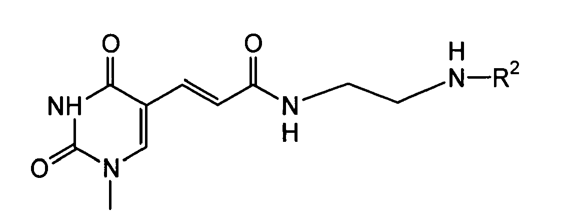 wherein R² represents a polyamine such as spermine, spermidine, glucosamine, and galactosamine,
is introduced thereinto.

The siRNA localized in the cytoplasm of the present invention can be obtained by forming a double-strand according to a routine method by use of the thus-produced sense strand or antisense strand having the chemical modification group introduced therein, or both.

This formation of the double-strand can be performed by dissolving the unmodified sense strand and the antisense strand with introduction of the chemical modification group, the antisense strand with introduction of the chemical modification group and the unmodified antisense strand, or the sense strand with introduction of the chemical modification group and the antisense strand with introduction of the chemical modification group in a polar organic solvent such as acetonitrile, dimethylformamide, dimethylacetamide, and dimethylsulfoxide, followed by stirring for 1 to 10 hours. In this procedure, the reaction solution can be heated to a higher temperature for promoting the reaction, for example, 30 to 60 °C, if desired.

The siRNA localized in the cytoplasm thus obtained is purified according to a routine method by using reverse-phase high-performance liquid chromatography or the like.

### Best Mode for Carrying Out the Invention

Next, the best mode for carrying out the present invention will be described with reference to Examples.

Cytoplasmic localization, enzymatic degradation resistance, degradation resistance in serum, telomerase inhibition activity, and tyrosine kinase activity inhibition in each Example are evaluated as follows.

### (1) Cytoplasmic localization:

A fluorescently labeled, modified DNA is suspended at a concentration of 1 µM in a physiological saline to prepare a sample. Separately, leukemia cells (Jurkat) are added at a concentration of 10⁶ cells/ml to a standard nutrient medium, to which the suspension of the modified DNA is in turn added, and cultured for 24 hours under conditions of 5% CO₂ and 37 °C. After culture, the cells are centrifuged, then washed three times with a PBS (-) buffer solution, and evaluated by use of flow cytometry (manufactured by BECKMAN COULTER, product code: "Epics XL") and a fluorescence and laser scanning confocal microscope (manufactured by BIO-RAD, product code: "Radiance 2000").

### (2) Enzymatic degradation resistance:

A nutrient medium containing a modified DNA at a concentration of 1 µM is supplemented with 100 units of an enzyme (DNase 1) and cultured at 37 °C for 10 minutes, followed by analysis by RP-HPLC to determine the degradation rate of the modified DNA.

### (3) Degradation resistance in serum:

A modified DNA with a concentration of 1 µM and fetal bovine serum (FBS) with a concentration of 10% are added into a nutrient medium and cultured at 37 °C for 2 hours, followed by analysis by RP-HPLC to determine the degradation rate of the modified DNA.

### (4) Telomerase inhibition activity:

A modified DNA is added at a concentration of 1 µM to an RPMI medium containing leukemia cells (Jurkat) at a concentration of 1x10⁶ cells/ml and cultured at 37 °C for 48 hours under conditions of 5% CO₂ and 37 °C to determine telomerase activity inhibition in terms of IC₅₀ (nM) by TRAP assay.

### (5) Tyrosine kinase activity inhibition:

A sample is added at a concentration of 5 µM to leukemia cells K-562 at a cell concentration of 1x10⁶ cells/ml and cultured for 48 hours under conditions of 5% CO₂ and 37 °C to determine its inhibition rate by protein tyrosine kinase assay.

### Example 1

An automatic DNA synthesizer (manufactured by Cruachem, product name: "PS250") was used to chemically modify the 5'-terminus of an oligonucleotide HIV-1 Rev (5'-SEQ ID NO: 16 in Sequence Listing-3') with an O-aminoethoxyethyl-O'-cyanoethylphosphoric ester residue on a CPG support according to a routine method.

Subsequently, the oligonucleotide was supplemented and reacted at 20 °C for 5 hours with 0.5 M solution prepared by dissolving hexamethylene diisocyanate in acetonitrile and then reacted with a peptide fragment HIV-1 Rev (SEQ ID NO: 1 in Sequence Listing) having a free N-terminal amino group with the protected amino acid side chain, thereby binding the NES peptide to the 5'-terminus of the oligonucleotide via the hexamethylene diisocyanate.

Next, this reaction product was supplemented with ammonia water with a concentration of 28% and stirred at 55 °C for 5 hours, thereby cleaving the produced conjugate from the solid support and removing the protecting group from the peptide.

A DNA localized in the cytoplasm represented by the formula
3'-SEQ ID NO: 16 in Sequence Listing-5'-O-CO-NH-CH₂CH₂NH-CO-NH-(CH₂)₆-NH-SEQ ID NO: 1 in Sequence Listing,
wherein the first Ala in SEQ ID NO: 1 in Sequence Listing is β-alanine,
was thus obtained at a yield of 10.7%.

The enzymatic degradation resistance of the DNA localized in the cytoplasm thus obtained was 29.1%, the degradation resistance in serum thereof was 42.3%, the telomerase inhibition activity thereof was 120 nM, and the tyrosine kinase activity inhibition thereof was approximately 50%. The enzymatic degradation resistance of the raw material DNA used as a control was 49.2%, the degradation resistance in serum thereof was 56.9%, the telomerase inhibition activity thereof was 40 nM, and the tyrosine kinase activity inhibition thereof was approximately 25%. The heat of fusion between the DNA localized in the cytoplasm and its complementary DNA or RNA was almost the same as the melting point of the raw material DNA used as a control.

Next, the DNA localized in the cytoplasm thus obtained was dissolved in acetonitrile and supplemented and reacted with an equimolar amount of fluorescein isothiocyanate to fluorescently label the DNA localized in the cytoplasm.

This fluorescently labeled DNA localized in the cytoplasm was examined for its cytoplasmic localization. For comparison, the fluorescently labeled raw material DNA used as a control was also examined for its cytoplasmic localization. When they were compared, the former was shown to have higher cytoplasmic localization.

### Example 2

A DNA localized in the cytoplasm represented by the formula 5'-SEQ ID NO: 17 in Sequence Listing-3'-PO(OH)-O-CH₂CH₂OCH₂CH₂-NH-CO-SEQ ID NO: 1 in Sequence listing,
wherein the first Ala in SEQ ID NO: 1 in Sequence Listing is β-alanine, was obtained at a yield of 2.7% in the same way as in Example 1 except that 5'-SEQ ID NO: 17 in Sequence Listing-3' was used as a DNA to introduce the NES peptide (SEQ ID NO: 1 in Sequence Listing) via a residue as a linker represented by the formula 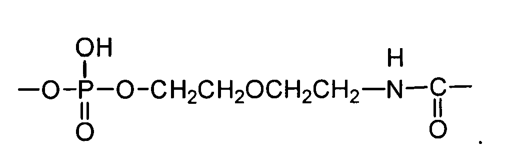 The enzymatic degradation resistance thereof was 29.1 %, the degradation property in serum was 42.3%, and the telomerase inhibition activity in a system using a cell lysis solution was 120 nM. In a cell system, approximately 12% telomerase activity inhibition was confirmed.

The observed telomerase inhibition activity of the raw material DNA used as a control was 400 nM or higher in a non-cell system and 0% in a cell system.

Next, this DNA localized in the cytoplasm was fluorescently labeled in the same way as in Example 1 and examined for its cytoplasmic localization. For comparison, the fluorescently labeled raw material DNA used as a control was also examined for its cytoplasmic localization. When they were compared, the former was shown to have higher cytoplasmic localization.

### Example 3

A DNA localized in the cytoplasm represented by the formula
5'-SEQ ID NO: 18 in Sequence Listing-3'-PO(OH)-O-CH₂CH₂OCH₂CH₂-NH-CO-SEQ ID NO: 3 in Sequence listing,
wherein the first Ala in SEQ ID NO: 1 in Sequence Listing is β-alanine, was obtained in the same way as in Example 2 except that 5'-SEQ ID NO: 18 in Sequence Listing-3' and MAPKK (SEQ ID NO: 3 in Sequence Listing) were used as a DNA and an NES peptide, respectively.

The enzymatic degradation resistance thereof was 34.2%, the degradation resistance in serum was 41.4%, and tyrosine kinase activity inhibition was approximately 46.2%. The enzymatic degradation resistance of the raw material DNA used as a control was 49.2%, the degradation resistance in serum thereof was 56.9%, and the tyrosine kinase activity inhibition thereof was approximately 21.8%.

Next, this DNA localized in the cytoplasm was fluorescently labeled in the same way as in Example 1 and examined for its cytoplasmic localization. For comparison, the raw material DNA used as a control was also examined for its cytoplasmic localization.

When they were compared, the DNA localized in the cytoplasm of the present invention exhibited cytoplasmic localization. Moreover, the DNA localized in the cytoplasm was shown to have more excellent enzymatic degradation resistance, degradation resistance in serum, and tyrosine kinase inhibition activity than those exhibited by the raw material DNA.

### Comparative Example

A DNA modified with an NLS peptide SV40 T antigen (SEQ ID NO: 12 in Sequence Listing) used instead of the NES peptide HIV-1 Rev in Example 1 in the same way as above was fluorescently labeled and examined for its cytoplasmic localization. As a result, its photomicrograph was exactly the same as that of the raw material DNA, wherein no cytoplasmic localization was observed.

### Production Example 1

An automatic DNA/RNA synthesizer (manufactured by Cruachem, product name: "PS250") was used to chemically modify the 5'-terminus of the sense strand (5'-SEQ ID NO: 1 in Sequence Listing-3') (hereinafter, referred to as RNA₁) or the antisense strand (5'-SEQ ID NO: 2 in Sequence Listing-3') (hereinafter, referred to as RNA₂) of an RNA with an aminating reagent (manufactured by Glen Research, product name: "5'-Amino Modifier 5") on controlled pore glass (hereinafter, referred to as CPG) by a solid phase method according to a routine method.

Subsequently, the obtained reaction product was supplemented with ammonia water with a concentration of 28% and stirred at 50 °C for 6 hours, thereby cleaving the chemically modified product from the solid support and removing the protecting group, followed by purification by reverse-phase high-performance liquid chromatography. The obtained chemically modified RNA was identified by mass spectrometry (MALDI TOF-MS). The following RNAs of two types having the chemically modified terminus were thus produced:
RNA₃ 5'-SEQ ID NO: 19 in Sequence Listing-3' (sense); and
RNA₄ 5'-SEQ ID NO: 20 in Sequence Listing-3' (antisense),
wherein n=-O-CH₂CH₂-O-CH₂CH₂NH₂ in the nucleotide sequences.

Results of MALDI TOF-MS of the RNAs thus obtained are shown in Table 5.

### Production Example 2

An aminating reagent for substitution at intermediate positions (manufactured by Glen Research, product name: "Amino Modifier C₂dT") was used to produce, in the same way as in Production Example 1, the following RNAs of four types in which t or u located at a non-terminal position in the nucleotide sequence was substituted by X:
RNA₅ 5'-SEQ ID NO: 21 in Sequence Listing-3' (sense);
RNA₆ 5'-SEQ ID NO: 22 in Sequence Listing-3' (antisense);
RNA₇ 5'-SEQ ID NO: 23 in Sequence Listing-3' (sense); and
RNA₈ 5'-SEQ ID NO: 24 in Sequence Listing-3' (antisense), wherein

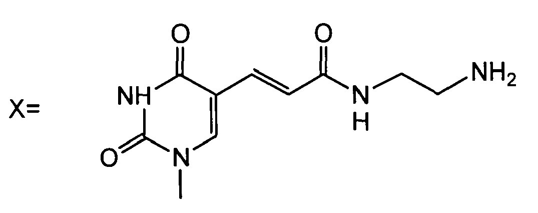

Results of MALDI TOF-MS of the RNAs thus obtained are shown in Table 5.

### Production Example 3

The 5'-terminus of the RNA₁ was chemically modified in the same way as in Production Example 1. The monomethoxytrityl (MMT) group, a protecting group for a terminal amino group, was treated for 1 minute with a solution of 3% trichloroacetic acid acetonitrile and thereby removed.

Subsequently, the RNA₁ was supplemented and reacted at 20°C for 5 hours with 0.5 M solution prepared by dissolving hexamethoxy diisocyanate in acetonitrile, to remove a product, which was in turn sequentially reacted in dimethylformamide with polyamines or saccharides of various types or peptides of various types having a free amino group.

Next, this reaction product was treated at 50 °C for 6 hours in concentrated ammonia water according to a routine method, thereby achieving cleavage from the solid support and the removal of the protecting group. The obtained 5'-terminal conjugate RNA was purified by reverse-phase high-performance liquid chromatography and identified by MALDI TOF-MS.

The RNAs shown in Table 1 in which the polyamine, saccharide, or peptide was introduced into the 5'-terminus were thus obtained.

Results of MALDI TOF-MS of the RNAs thus obtained are shown in Table 5.
Sense strand 5'-SEQ ID NO: 25 in Sequence Listing-3',
wherein n=-O-CH₂CH₂-O-CH₂CH₂-NH-R¹ in the nucleotide sequence.

**Table 1**

| Type of RNA | R¹ | Sequence of amino acid in R¹ |
|---|---|---|
| RNA₉ | spermine | - |
| RNA₁₀ | spermidine | - |
| RNA₁₁ | tris(2-amioethyl)amine | - |
| RNA₁₂ | triethyltetramine | - |
| RNA₁₃ | glucosamine | - |
| RNA₁₄ | galactosamine | - |
| RNA₁₅ | HIV-1 Rev nuclear export signal peptide | SEQ ID NO: 1 in Sequence Listing |
| RNA₁₆ | PKIα nuclear export signal peptide | SEQ ID NO: 2 in Sequence Listing |
| RNA₁₇ | MAPKK nuclear export signal peptide | SEQ ID NO: 3 in Sequence Listing |
| RNA₁₈ | Dsk-1 nuclear export signal peptide | SEQ ID NO: 4 in Sequence Listing |
| RNA₁₉ | TFIII A nuclear export signal peptide | SEQ ID NO: 11 in Sequence Listing |
| RNA₂₀ RNA₂₀ | HIV-1 tat C-terminal membrane fusion peptide | SEQ ID NO: 5 in Sequence Listing |
| RNA₂₁ | gp-41 membrane fusion peptide | SEQ ID NO: 6 in Sequence Listing |
| RNA₂₂ | SV40 T antigen nuclear localization signal peptide | SEQ ID NO: 12 in Sequence Listing |
| RNA₂₃ | HIV-1 tat nuclear localization signal peptide | SEQ ID NO: 13 in Sequence Listing |
| RNA₂₄ | artificially designed amphipathic α-helical peptide | SEQ ID NO: 7 in Sequence Listing |
| RNA₂₅ | artificially designed amphipathic β-sheet peptide | SEQ ID NO: 8 in Sequence Listing |
| RNA₂₆ | artificially designed amphipathic β-sheet peptide | SEQ ID NO: 14 in Sequence Listing |
| RNA₂₇ | artificially designed amphipathic β-sheet peptide | SEQ ID NO: 15 in Sequence Listing |

### Production Example 4

A sense strand containing a chemical modification group X at a non-terminal position was produced in the same way as in Production Example 2. The trifluoroacetyl group, a protecting group of the aminating reagent, was treated for 1 minute with 20% acetonitrile solution of ethylene glycol and thereby removed. Subsequently, the RNA was reacted with an acetonitrile solution of hexamethylene diisocyanate in the same way as in Production Example 3 and then with dimethylformamide solution of polyamines of various types and treated in the same way as in Production Example 3, thereby obtaining RNAs shown in Table 2 in which the chemical modification group Y was introduced at the non-terminal position.

Results of MALDI TOF-MS of the RNAs thus obtained are shown in Table 5.
Sense strand=5'-SEQ ID NO: 26 in Sequence Listing-3'.

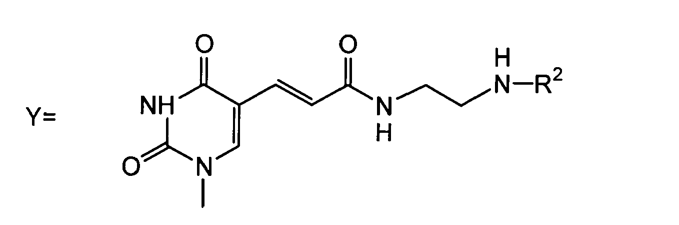

**Table 2**

| Type of RNA | R² |
|---|---|
| RNA₂₈ | spermine |
| RNA₂₉ | spermidine |
| RNA₃₀ | glucosamine |
| RNA₃₁ | galactosamine |

These sense strands and the antisense strands having the chemical modification group Y at the non-terminal position obtained in Production Example 3 were used to produce siRNAs localized in the cytoplasm. The siRNAs localized in the cytoplasm thus obtained were identified by MALDI TOF-MS.

### Production Example 5

Production Examples 3 and 4 were combined to produce RNAs shown in Table 3 in which the 5'-terminus of the RNA₁ was chemically modified with n and in which t located at a non-terminal position of the nucleotide sequence was substituted by X. Results of MALDI TOF-MS of the conjugate RNAs thus obtained are shown in Table 5.
Sense strand=5'- SEQ ID NO: 27 in Sequence Listing-3',
wherein n=-O-CH₂CH₂-O-CH₂CH₂-NH-R¹ in the nucleotide sequence.

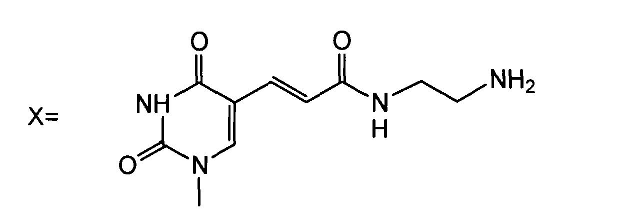

**Table 3**

| Type of RNA | R¹ | Sequence of amino acid in R¹ |
|---|---|---|
| RNA₃₂ | spermine | - |
| RNA₃₃ | spermidine | - |
| RNA₃₄ | glucosamine | - |
| RNA₃₅ | galactosamine | - |
| RNA₃₆ | HIV-1 Rev nuclear export signal peptide | SEQ ID NO: 1 in Sequence Listing |
| RNA₃₇ | PKIα nuclear export signal peptide | SEQ ID NO: 2 in Sequence Listing |
| RNA₃₈ | MAPKK nuclear export signal peptide | SEQ ID NO: 3 in Sequence Listing |
| RNA₃₉ | Dsk-1 nuclear export signal peptide | SEQ ID NO: 4 in Sequence Listing |
| RNA₄₀ | HIV-1 tat C-terminal membrane fusion peptide | SEQ ID NO: 5 in Sequence Listing |
| RNA₄₁ | gp-41 membrane fusion peptide | SEQ ID NO:6 in Sequence Listing |
| RNA₄₂ | SV40 T antigen nuclear localization signal peptide | SEQ ID NO: 12 in Sequence Listing |
| RNA₄₃ | artificially designed amphipathic α-helical peptide | SEQ ID NO: 7 in Sequence Listing |
| RNA₄₄ | artificially designed amphipathic β-sheet peptide | SEQ ID NO: 14 in Sequence Listing |

### Production Example 6

RNAs shown in Table 4 in which the 5'-terminus of the RNA₁ was chemically modified with n and in which a plurality of u or t located at non-terminal positions of the nucleotide sequence was substituted by X were produced in the same way as in Production Example 5. Results of MALDI TOF-MS of the conjugate RNAs thus obtained are shown in Table 5.

**Table 4**

| Sense strand | SEQ ID NO: 28 in Sequence Listing |
|---|---|
| n at 5'-terminus | -O-CH₂CH₂-O-CH₂CH₂NH-R¹ |
| X | 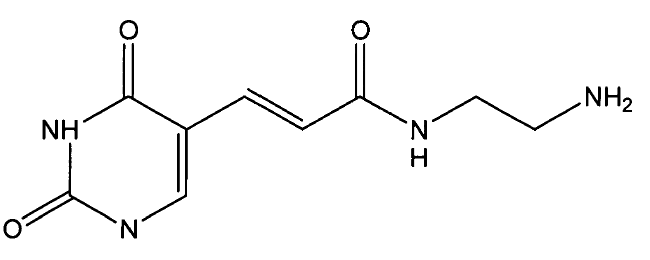 |
| RNA₄₅ | R¹=HIV-1 Rev nuclear export signal peptide Sequence of amino acid: SEQ ID NO: 1 in Sequence Listing |
| RNA₄₆ | R¹=PKIα nuclear export signal peptide Sequence of amino acid: SEQ ID NO: 2 in Sequence Listing |
| RNA₄₇ | R¹=MAPKK nuclear export signal peptide Sequence of amino acid: SEQ ID NO: 3 in Sequence Listing |

**Table 5**

| Type of RNA | Ms (calculated value) | MALDI TOF-MS |
|---|---|---|
| RNA₃ | 6739.11 | 6740.34 |
| RNA₄ | 6910.15 | 6911.72 |
| RNA₅ | 6670.13 | 6672.21 |
| RNA₆ | 6742.10 | 6740.68 |
| RNA₇ | 6768.17 | 6766.96 |
| RNA₈ | 7036.24 | 7037.33 |
| RNA₉ | 7110.56 | 7115.64 |
| RNA₁₀ | 7053.42 | 7058.35 |
| RNA₁₁ | 7054.45 | 7056.04 |
| RNA₁₂ | 7054.46 | 7059.68 |
| RNA₁₃ | 7088.87 | 7070.43 |
| RNA₁₄ | 7088.87 | 7078.43 |
| RNA₁₅ | 8187.79 | 8190.78 |
| RNA₁₆ | 8290.96 | 8294.23 |
| RNA₁₇ | 8694.27 | 8690.33 |
| RNA₁₈ | 8512.01 | 8514.80 |
| RNA₁₉ | 9185.87 | 9186.01 |
| RNA₂₀ | 8593.04 | 8595.87 |
| RNA₂₁ | 8528.13 | 8534.35 |
| RNA₂₂ | 8019.64 | 8032.56 |
| RNA₂₃ | 8912.59 | 8910.92 |
| RNA₂₄ | 8515.27 | 8516.70 |
| RNA₂₅ | 8501.25 | 8503.23 |
| RNA₂₆ | 9039.95 | 9041.20 |
| RNA₂₇ | 8843.85 | 8847.54 |
| RNA₂₈ | 6899.47 | 6900.08 |
| RNA₂₉ | 6842.33 | 6845.43 |
| RNA₃₀ | 6876.78 | 6877.03 |
| RNA₃₁ | 6876.78 | 6877.55 |
| RNA₃₂ | 7208.61 | 7212.33 |
| RNA₃₃ | 7151.47 | 7153.67 |
| RNA₃₄ | 7186.92 | 7169.98 |
| RNA₃₅ | 7186.92 | 7172.32 |
| RNA₃₆ | 8285.84 | 8288.55 |
| RNA₃₇ | 8389.01 | 8390.76 |
| RNA₃₈ | 8792.31 | 8793.22 |
| RNA₃₉ | 8610.05 | 8612.01 |
| RNA₄₀ | 8691.09 | 8690.44 |
| RNA₄₁ | 8626.18 | 8629.23 |
| RNA₄₂ | 8117.69 | 8118.02 |
| RNA₄₃ | 8613.32 | 8620.46 |
| RNA₄₄ | 9138.00 | 9135.67 |
| RNA₄₅ | 8579.99 | 8580.85 |
| RNA₄₆ | 8683.16 | 8688.04 |
| RNA₄₇ | 9086.46 | 9090.11 |

### Examples 4 to 17

siRNAs localized in the cytoplasm were formed by using the RNA₉ and RNA₁₅ to RNA₂₇ as sense strands and the RNA₂ as an antisense strand.

A 1-µM portion each of the siRNAs localized in the cytoplasm thus obtained was added to an RPMI medium containing 10% by mass of fetal bovine serum (FBS) and incubated at 37 °C to measure the degradation rates of the conjugate siRNAs after a lapse of 2, 4, 6, 12, and 24 hours. This measurement was performed by separating the siRNA by electrophoresis of 20% by mass of polyacrylamide gel and detecting it by use of a silver impregnation method. This result is shown in Table 6.

The degradation rate of an siRNA formed with the sense strand RNA₁ having no chemical modification group introduced and the antisense strand RNA₂ having no chemical modification group introduced was also written as a control.

**Table 6**

| Exampl e | siRNA (sense/antisense) | Degradation rate of siRNA, % | | | | |
|---|---|---|---|---|---|---|
| | | after 2 hours | after 4 hours | after 6 hours | after 12 hours | after 24 hours |
| Control | RNA₁/RNA₂ | 65.2 | 82.0 | 100 | 100 | 100 |
| 4 | RNA₉/RNA₂ | 47.1 | 62.6 | 76.7 | 87.7 | 100 |
| 5 | RNA₁₅/RNA₂ | 6.6 | 20.9 | 27.9 | 36.5 | 45.2 |
| 6 | RNA₁₆/RNA₂ | 8.0 | 13.7 | 21.6 | 26.8 | 35.2 |
| 7 | RNA₁₇/RNA₂ | 7.8 | 26.8 | 37.6 | 39.8 | 53.8 |
| 8 | RNA₁₈/RNA₂ | 2.6 | 16.1 | 21.6 | 26.8 | 41.7 |
| 9 | RNA₁₉/RNA₂ | 12.7 | 24.0 | 32.0 | 42.6 | 48.4 |
| 10 | RNA₂₀/RNA₂ | 9.3 | 19.1 | 22.9 | 27.3 | 33.4 |
| 11 | RNA₂₁/RNA₂ | 5.1 | 17.5 | 31.7 | 32.7 | 36.8 |
| 12 | RNA₂₂/RNA₂ | 3.6 | 9.3 | 15.6 | 20.1 | 28.9 |
| 13 | RNA₂₃/RNA₂ | 5.8 | 11.4 | 14.1 | 20.3 | 25.9 |
| 14 | RNA₂₄/RNA₂ | 7.9 | 14.7 | 19.4 | 28.4 | 35.9 |
| 15 | RNA₂₅/RNA₂ | 6.0 | 11.7 | 15.6 | 18.9 | 24.1 |
| 16 | RNA₂₆/RNA₂ | 1.6 | 5.7 | 9.8 | 14.7 | 17.4 |
| 17 | RNA₂₇/RNA₂ | 4.4 | 10.7 | 14.1 | 16.1 | 17.8 |

As can be seen from this table, the siRNAs using the sense strand having the chemical modification group introduced at the 5'-terminus and the antisense strand having the chemical modification group introduced at the 5'-terminus obviously exhibited considerably improved resistance to enzymes except that the siRNA using the sense strand of the spermine conjugate (RNA₉) had low resistance. Particularly, those using the sense strand having seven LeuArg or LeuLys sequences conjugated (RNA₂₆ or RNA₂₇) exhibited high resistance.

### Examples 18 to 22

siRNAs were formed in the same way as in Examples 4 to 17 from the sense strands having the chemical modification group at the non-terminal position and the antisense strand having no chemical modification group to measure their degradation rates by enzymes. This result is shown in Table 7.

**Table 7**

| Example | siRNA (sense/antisense) | Degradation rate of siRNA, % | | | | |
|---|---|---|---|---|---|---|
| | | after 2 hours | after 4 hours | after 6 hours | after 12 hours | after 24 hours |
| 18 | RNA₅/RNA₂ | 36.2 | 71.3 | 81.3 | 94.5 | 100 |
| 19 | RNA₂₈/RNA₂ | 19.9 | 30.5 | 40.7 | 45.7 | 53.3 |
| 20 | RNA₂₉/RNA₂ | 12.9 | 19.5 | 25.7 | 37.7 | 42.9 |
| 21 | RNA₃₀/RNA₂ | 7.8 | 16.5 | 22.2 | 37.7 | 40.0 |
| 22 | RNA₃₁/RNA₂ | 11.9 | 27.8 | 37.6 | 42.9 | 48.7 |

Most of the siRNAs formed from the sense strands having the chemical modification group at the non-terminal position and the antisense strand having no chemical modification group had 50% or lower degradation rates even after a lapse of 24 hours.

### Examples 23 to 35

siRNAs were formed in the same way as in Examples 4 to 17 from the sense strands having the chemical modification groups simultaneously introduced at the 5'-terminus and the non-terminal position(s) and the antisense strand having no chemical modification group to measure their degradation rates by enzymes. This result is shown in Table 8.

**Table 8**

| Example | siRNA (sense/antisense) | Degradation rate of siRNA, % | | | | |
|---|---|---|---|---|---|---|
| | | after 2 hours | after 4 hours | after 6 hours | after 12 hours | after 24 hours |
| 23 | RNA₃₂/RNA₂ | 25.1 | 38.8 | 43.7 | 52.0 | 62.0 |
| 24 | RNA₃₆/RNA₂ | 11.7 | 14.6 | 19.0 | 23.2 | 28.2 |
| 25 | RNA₃₇/RNA₂ | 5.9 | 12.0 | 15.8 | 18.6 | 23.8 |
| 26 | RNA₃₈/RNA₂ | 8.3 | 17.5 | 23.2 | 30.6 | 39.6 |
| 27 | RNA₃₉/RNA₂ | 3.6 | 9.1 | 13.8 | 16.1 | 24.7 |
| 28 | RNA₄₀/RNA₂ | 13.1 | 14.7 | 28.5 | 33.6 | 43.9 |
| 29 | RNA₄₁/RNA₂ | 16.9 | 21.0 | 24.8 | 32.3 | 43.3 |
| 30 | RNA₄₂/RNA₂ | 3.0 | 6.5 | 8.8 | 13.3 | 22.9 |
| 31 | RNA₄₃/RNA₂ | 1.5 | 12.0 | 13.7 | 15.9 | 20.5 |
| 32 | RNA₄₄/RNA₂ | 0.9 | 7.4 | 9.3 | 12.7 | 14.4 |
| 33 | RNA₄₅/RNA₂ | 0 | 2.4 | 2.8 | 4.4 | 5.4 |
| 34 | RNA₄₆/RNA₂ | 0 | 0.7 | 2.4 | 3.7 | 5.1 |
| 35 | RNA₄₇/RNA₂ | 0.7 | 2.1 | 2.8 | 4.0 | 4.8 |

As can be seen from this table, the siRNAs using the sense strands having the chemical modification groups simultaneously introduced at the 5'-terminus and the non-terminal position exhibited high enzymatic degradation resistance particularly when conjugated with the PKIα nuclear export signal peptide (RNA₃₇), Dsk-1 nuclear export signal peptide (RNA₃₉), SV40 T antigen nuclear localization signal peptide (RNA₄₂), artificial peptide (RNA₄₃), and artificial peptide (RNA₄₄).

Moreover, all the siRNA simultaneously conjugated with the chemical modification groups at the 5'-terminus and a plurality of non-terminal positions exhibited high resistance such as 5% or lower degradation rate when conjugated with the HIV-1 Rev nuclear export signal peptide (RNA₄₅), PKIα nuclear export signal peptide (RNA₄₆), and MAPKK nuclear export signal peptide (RNA₄₇).

### Reference Example 1

Leukemia cells (Jurkat: 0.5x10⁶ cells/ml) was added to a 100-µmM portion each of the siRNAs obtained in Examples 4 to 35 and incubated at 37 °C for 48 hours in an atmosphere containing 5% by volume of CO₂ in an RPMI medium containing 10% by mass of FBS to measure cytotoxicity by use of a cell viability kit (manufactured by Promega).

As a result, slight cytotoxicity as much as 90% cell viability after 48 hours was observed in the siRNAs obtained Examples 24 and 29, whereas almost no cytotoxicity was observed in the remaining siRNAs.

### Reference Example 2

The fluorescently labeled conjugate siRNAs (1 µM each) were added to leukemia cells (Jurkat: 1x10⁶ cells/ml) and incubated at 37 °C for 48 hours in the presence of 5% CO₂ in an RPMI medium containing 10% FBS. Then, the cells were washed three times with PBS (-) and observed for their introduction into the cells and intracellular localization by use of a fluorescence and laser scanning confocal microscope.

As a result, cellular uptake was significantly promoted in all the siRNAs obtained in Examples 5, 6, 7, 8, 12, 13, 14, and 17, which were conjugated with the peptides of various types at the 5'-terminus of the sense strand (under observation with the confocal laser induced fluorescence microscope).

The siRNAs obtained in Examples 5, 6, 7, and 8, which were conjugated with the nuclear export signal peptide, and the siRNA obtained in Example 14, which was conjugated with the artificial peptide, were shown to be respectively localized in the cytoplasm, whereas the siRNAs obtained in Examples 12 and 13, which were conjugated with the nuclear export signal peptide, and the siRNA obtained in Example 16, which was conjugated with the artificial peptide, were shown to be respectively localized in the nucleus.

The conjugate siRNAs (100 nM each) were added to leukemia cells (K-562: 1x10⁶ cells/ml) and incubated at 37 °C for 48 hours in the presence of 5% CO₂ in an RPMI medium containing 10% FBS. Subsequently, their tyrosine kinase inhibition activities were measured by protein tyrosine kinase assay to indicate them in terms of percentage.

As a result, only 6% inhibition effect was observed in the natural siRNA (RNA₁/RNA₂), whereas inhibition effects as high as 90% or more were observed in the conjugate siRNAs. Particularly the siRNA (RNA₃₆/RNA₂) obtained in Example 24, which was simultaneously conjugated with the HIV-1 Rev export signal peptides in the 5'-terminus and the proximity of the 3'-terminus of the sense strand, achieved almost 100% inhibition effect.

### Industrial Applicability

According to the present invention, efficacy of genetic medicine can be improved. Therefore, the present invention is highly usable in medical fields.

## Claims

1. A DNA or RNA localized in the cytoplasm having the 3'-terminus or 5'-terminus chemically modified with a group represented by the formula
-PO(OH)-O-CH₂CH₂OCH₂CH₂NH-CO-peptide or
-O-CO-NH-CH₂CH₂NHCONH(CH₂)₆NH-CO-NH-peptide.

2. A DNA or RNA localized in the cytoplasm modified with a peptide that is a signal peptide selected from HIV-1 Rev (SEQ ID NO: 1 in Sequence Listing), PKIα (SEQ ID NO: 2 in Sequence Listing), MAPKK (SEQ ID NO: 3 in Sequence Listing), and Dsk-1 (SEQ ID NO: 4 in Sequence Listing), or a membrane fusion peptide selected form an HIV-1 tat C-terminal membrane fusion peptide (SEQ ID NO: 5 in Sequence Listing), a gp-41 membrane fusion peptide (SEQ ID NO: 6 in Sequence Listing), an artificially designed amphipathic α-helical peptide (SEQ ID NO: 7 in Sequence Listing), and an artificially designed amphipathic β-sheet peptide (SEQ ID NO: 8, 14, or 15 in Sequence Listing).

3. The DNA localized in the cytoplasm according to claim 1 or 2, wherein the DNA is an oligo DNA that exhibits a genetic medicinal activity.

4. The RNA localized in the cytoplasm according to claim 1 or 2, wherein the RNA is an oligo RNA that exhibits a genetic medicinal activity.

5. A telomerase inhibitor comprising a DNA or RNA localized in the cytoplasm according to any one of claims 1 to 4 as an active ingredient.

6. A tyrosine kinase inhibitor comprising a DNA or RNA localized in the cytoplasm according to any one of claims 1 to 4 as an active ingredient.

7. A therapeutic agent for leukemia comprising a DNA or RNA localized in the cytoplasm according to any one of claims 1 to 4 as an active ingredient.

8. A cell growth inhibitor comprising a DNA or RNA localized in the cytoplasm according to any one of claims 1 to 4 as an active ingredient.

9. A siRNA localized in the cytoplasm **characterized in that** a chemical modification group is introduced into the 5'-terminus of at least one of the sense strand and the antisense strand constituting the double-strand or a dangling end of the antisense strand, or both.

10. A siRNA localized in the cytoplasm **characterized in that** at least one of the sense strand and the antisense strand contains a chemical modification group at a non-terminal position.

11. The siRNA localized in the cytoplasm according to claim 9 or 10, wherein the chemical modification group has a polyamine molecule bonded thereto.

12. The siRNA localized in the cytoplasm according to claim 9 or 10, wherein the chemical modification group is a nuclear export signal peptide or membrane fusion peptide introduced via a bifunctional linker.

13. The siRNA localized in the cytoplasm according to claim 12, wherein the bifunctional linker is a residue of a divalent chemical modification group.
